# EUROPEAN PATENT APPLICATION

(11) **EP 3 345 498 A1**
(43) Date of publication of application: **11.07.2018**
(21) Application number: 17460003.1
(22) Date of filing: 08.02.2017
(51) Int. Cl.: A41B 11/12, A41F 11/16, A41B 9/12, A61F 13/08

(54) **THIGH BAND**

(30) Priority: 09.01.2017 PL 12591117
(71) Applicant: P.P.H.U LIDA Leszek WITKOWSKI, 95-070 Aleksandrow Lodzki (PL)
(72) Inventor: Leszek, Witkowski, 95-070 Aleksandrów Lódzki (PL)
(74) Representative: Dziubinska, Joanna

(57) **Abstract**

Thigh band is characterized in that the upper part (1) of the thigh band is in the form of an elastic with a various number of elements (2) holding the band on the leg, and this part (1) of the band is joined with the knit piece (4) by a seam (3) and gathered with a ribbing (5).

## Description

The subject of the utility model is a new and original thigh band, intended especially for women.

There are known various kinds of hosiery products intended for thighs or other parts of lower limbs.

From the patent description no. 187238 there is known "A correcting and compressing product, especially an elastic stocking or tricot", which has an elastic cuff on one side, covered with slip-resistant finish on the side in contact with the body.

From the American invention no. US2016/0167344 there is known a product in the form of a tubular knit piece gathered with lace on both ends.

From the American invention no. US2016/0000161 there is known a product in the form of a knit piece and at least to its one narrower side there is fixed a convex decorative element in the form of a flounce.

From the British invention no. GB2537869 there is known a hosiery product with decorative elements in upper parts of thighs.

Unlike products known from the state of the art, the solution according to the model protects thighs against rubbing and has decorative character. The solution according to the model may be manufactured from hosiery materials, or from materials used in sportswear.

The essence of the utility model consists in the fact that the upper part of the thigh band is in the form of an elastic with a various number of elements holding the band on the leg, and this part of the band is joined with the knit piece by a seam and gathered with a ribbing.

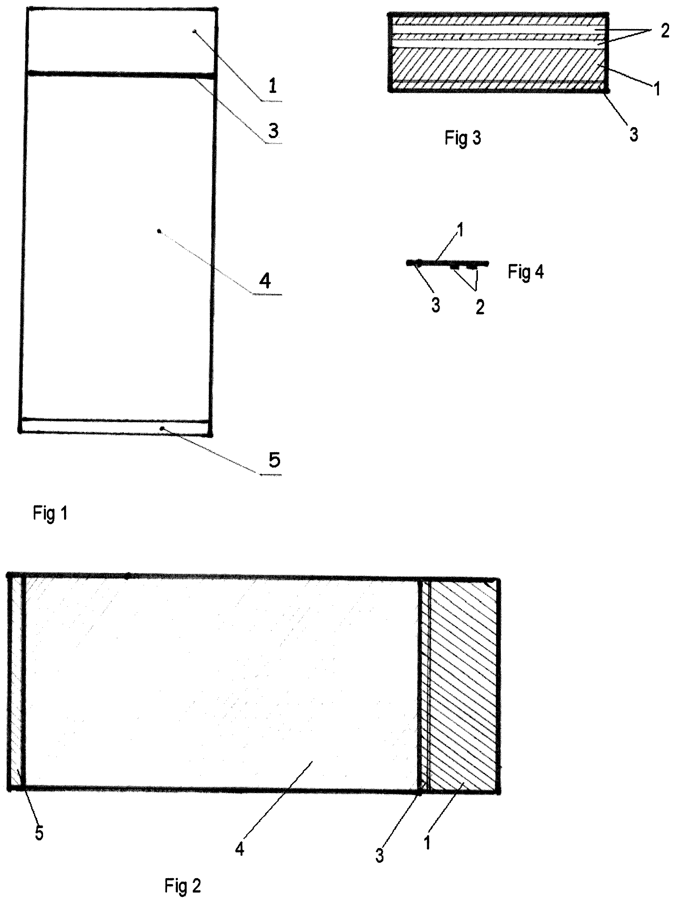

The band according to the model is presented on the enclosed figures, Fig. 1-4, showing the following:
Fig. 1 shows a front view of the whole band.
Fig. 2 shows a diversity of materials used for the band.
Fig. 3 shows a front view of a rear (bottom) side of the upper part of the band.
Fig. 4 shows a side view of an upper part of the band.

The thigh band according to the model consists of four elements permanently joined together. The upper part (1) of the band is in the form of an elastic or lace with a various number of elements, advantageously silicone straps that hold the band on the leg. The upper part (1) may be in the form of a stretch lace, or its inner surface (touching the leg) may be fully covered with a material sticking to the skin, advantageously silicone (2). That part (1) of the band is joined with the knit piece (4) by a seam (3). The knit piece (4) may be of any thickness, with or without any pattern. At the bottom the whole thigh band is finished with a ribbing (5), or it may be trimmed.

Three main elements of the thigh band: the upper part (1) of the band, the knit piece (4) and the ribbing (5), advantageously made from different materials. The seam (3) of the band may be covered with a lace sewn to it on the outside. The lace may be the same as the one making the upper part (1) of the band, or it may be a different lace or material. Both the upper part (1) of the band and the knit piece (4) may have additional elements of practical and decorative character, like small pockets and fasteners, and various decorative elements, like beads, sequins, or tabs. The seam (3) in the thigh band according to the model may be constructed in such a way that an additional part, e.g., a small pocket, may be sewn at its level from the underside in a permanent or mobile way. Such an additional part, e.g., a small pocket, sewn in the above way, is not visible during the ordinary use of the band, but it has a practical advantage as it is possible to put small things into it. It is particularly important when the band is intended for sport.

## Claims

1. Thigh band having knit piece,lace, silicone, ribbing is **characterized in that** the upper part (1) of the thigh band is in the form of an elastic with a various number of elements (2) holding the band on the leg, and this part (1) of the band is joined with the knit piece (4) by a seam (3) and gathered with a ribbing (5).

## Amended claims

### Amended claims in accordance with Rule 137(2) EPC.

1. Thigh band having knit piece,lace, silicone, ribbing is **characterized in that** an upper part (1) of the thigh band is in the form of an elastic with a various number of elements (2) holding the band on the leg, and below the upper part (1) of the band, there is a seam (3) that connects the upper part of the band with the knit, and the bottom part of the knitted piece (4) is gathered with a ribbing (5).
